# FASCICULE DE BREVET EUROPEEN

(11) **EP 0 690 050 B1**
(45) Date de publication et mention de la délivrance du brevet: **17.11.1999**
(21) Numéro de dépôt: 95401536.8
(22) Date de dépôt: 28.06.1995
(51) Int. Cl.: C07D 209/94, A61K 31/40, C07D 401/12, C07D 495/04

(54) **Dérivés (thia)cycloalkyl(b) indoles comme inhibiteurs de la 5-lipoxygénase**
(Thia)cycloalkyl(b)indolderivate als 5-lipoxygenase Inhibitoren
(Thia)cycloalkyl(b) indole derivatives as 5-lipoxygenase inhibitors

(30) Priorité: 28.06.1994 FR 9407888
(43) Date de publication de la demande: 03.01.1996
(73) Titulaire: ADIR ET COMPAGNIE, 92415 Courbevoie Cédex (FR)
(72) Inventeur: Caubere, Paul, F-54000 Nancy (FR); Jamart-Gregoire, Brigitte, F-54500 Vandoeuvre les Nancy (FR); Caubere, Catherine, F-54000 Nancy (FR); Manechez, Dominique, F-92800 Puteaux (FR); Renard, Pierre, F-78000 Versailles (FR); Adam, Gérard, F-78600 le Mesnil le Roi (FR); Nguyen, Catherine, F-67380 Lingolsheim (FR)

(56) Documents cités:
- EP-A- 0 304 156
- EP-A- 0 468 785
- EP-A- 0 635 491
- WO-A-91/06537
- WO-A-94/11378
- DE-A- 2 243 574
- JOURNAL OF THE CHEMICAL SOCIETY, PERKIN TRANSACTIONS 1, 1975 LETCHWORTH GB, pages 1280-1283, G. JONES ET AL. 'Some benzazocinecarboxylic acids ...'
- CHEMICAL ABSTRACTS, vol. 60, no. 5, 2 Mars 1964 Columbus, Ohio, US; abstract no. 5622g, L.A. AKSANOVA ET AL. 'Indole derivatives. XIV. Synthesis of 4-H-2,3,-dihydro-thieno(3,2-b) indoles.' & ZH. OBSHCH. KHIM. , vol. 34, no. 5, 1964 pages 1609-1613,
- JOURNAL OF MEDICINAL CHEMISTRY, vol. 36, no. 19, 17 Septembre 1993 WASHINGTON US, pages 2771-1787, J.H. HUTCHINSON ET AL. 'Substituted thiopyrano(2,3,4-c,d)indoles as...inhibitors of 5-lipoxyfenase.'
- JOURNAL OF MEDICINAL CHEMISTRY, vol. 35, no. 14, 10 Juillet 1992 WASHINGTON US, pages 2501-2524, J.H. MUSSER ET AL. '5-Lipoxygenase:...'

## Description

L'invention concerne de nouveaux dérivés (thia)cycloalkyl[b]indoles, leur procédé de préparation et les compositions pharmaceutiques qui les contiennent.

On connaît, comme art antérieur, les composés des demandes EP 468 785 et EP 502 106 qui sont décrits comme anti-inflammatoires, mais ceux-ci enseignent qu'une substitution sur la partie cyclo-alkyle est toujours nécessaire.

La demande EP 304 156 décrit par ailleurs des acides cyclohepta[b]indole alkanaoiques ayant des propriétés antagonistes de prostaglandines. On trouve également décrits dans l'art antérieur des composés cyclopenta[b]indoles (J.C.S. Perkin I, 1975 pp. 1280-1283 ; DE 2243574) et 2,3-dihydrothiéno[3,2-b]indoles (Chemical Abstracts 60 (5), 1964, 5622 g) utiles en tant que produts ou intermèdiaires de synthèse.

La demanderesse a découvert de puissants composés anti-inflammatoires dépourvus des effets secondaires (toxicité gastrointestinale) présentés par les inhibiteurs de cyclo-oxygénase actuellement sur le marché.

L'activité anti-inflammatoire des composés de l'invention permet au clinicien de disposer d'agents thérapeutiques utiles pour inhiber certains processus (et notamment l'activation de la 5-lipoxygénase) impliqués dans plusieurs pathologies à composante anti-inflammatoire (dont l'arthrite, la colite et le psoriasis) sans affecter la voie de la cyclo-oxygénase.

Les composés de l'invention permettent notamment une inhibition de la 5-lipoxygénase par un mécanisme anti-FLAP ("five lipoxygenase activatory protein"), inhibition qui s'avère être beaucoup plus élevée que celle des inhibiteurs redox ou des chélateurs de fer.

L'invention concerne plus particulièrement les composés de formule (I) : 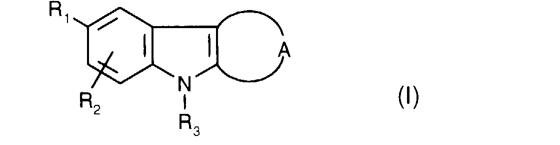 dans laquelle :
- R₁ représente un groupement Ar-(CH₂)ₙ-O- avec n représentant zéro ou un entier de 1 à 4, Ar étant un radical choisi parmi phényle, naphtyle, thiényle, furyle, pyrrolyle, imidazolyle, pyrazolyle, thiazolyle, isothiazolyle, oxazolyle, isoxazolyle, thiadiazole, oxadiazole, pyridyle, quinolyle, isoquinolyle, indolyle, benzofuryle, et benzothiényle ; Ar pouvant être non substitué ou substitué par un ou plusieurs radicaux choisis parmi halogène, alkyle, alkoxy, hydroxy et trifluorométhyle,
- R₂ représente un atome d'hydrogène ou un radical choisi parmi halogène, alkyle, alkoxy et trifluorométhyle,
- R₃ représente un radical choisi parmi hydrogène, alkyle, carboxyalkyle et alkoxycarbonylalkyle,
- A représente une chaîne alkylène -(CH₂)ₘ- avec m compris entre 3 et 6 ou un groupement de formule (α): 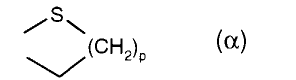 dans laquelle p est compris entre 1 et 4,
étant entendu que les termes "alkyle" et "alkoxy" désignent des groupements linéaires ou ramifiés de 1 à 6 atomes de carbone,
étant entendu que le composé de formule (I) ne peut représenter le 7-benzyloxy-1,2,3,4-tétrahydrocyclopenta[b]indole, le 7-benzyloxy-4*H*2,3-dihydrothiéno[3,2-b]indole, le 6-benzyloxy-1,2,3,4-tétrahydrocarbazole, ni le 6-benzyloxy-9-méthyl-1,2,3,4-tétrahydrocarbazole,
leurs isomères optiques, sous forme pure ou sous forme de mélange,
et leurs sels d'addition à un acide ou une base, pharmaceutiquement acceptables.

Parmi les bases pharmaceutiquement acceptables que l'on peut utiliser pour salifier les composés selon l'invention, on peut citer, à titre d'exemples et de façon non limitative, l'hydroxyde de sodium, l'hydroxyde de potassium, la triéthylamine, la diéthylamine, l'éthanolamine, l'arginine, la lysine, et la diéthanolamine.

Parmi les acides pharmaceutiquement acceptables que l'on peut utiliser pour salifier les composés selon l'invention, on peut citer, à titre d'exemples, et de façon non limitative, les acides chlorhydrique, sulfurique, phosphorique, tartrique, malique, fumarique, oxalique, méthane sulfonique, éthanesulfonique, camphorique et citrique.

L'invention s'étend également au procédé de préparation des composés de formule (I), caractérisé en ce que l'on condense, en présence d'un amidure de sodium ou sa combinaison avec un alcoolate (base complexe) telle que la base complexe amidure de sodium-tertiobutylate de sodium (NaNH₂-tBuONa), le composé de formule (II) : 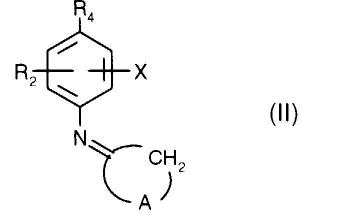 dans laquelle R₂ et A sont tels que définis dans la formule (I), X représente un atome d'halogène et R₄ représente un radical alkoxy, ou tétrahydropyranyloxy, pour obtenir :
- soit un composé de formule (III) : 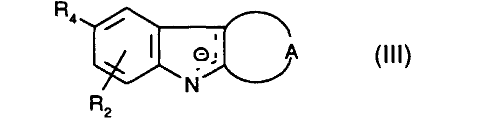 dans laquelle R₂, R₄ et A sont tels que définis précédemment,
- soit, après hydrolyse, un composé de formule (IV) : 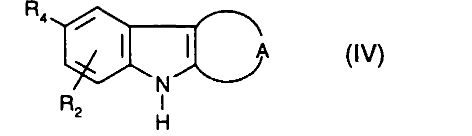 dans laquelle R₂, R₄ et A sont tels que définis précédemment,
composé de formule (III), ou composé de formule (IV) sous forme de son sel alcalin, qui est substitué sur l'azote indolique par un radical de formule R₃', dans lequel R₃' a la même signification que R₃ tel que défini dans la formule (I) à l'exception de l'hydrogène, pour obtenir un composé de formule (V) : 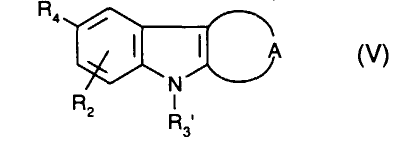 dans laquelle R₂, R₄, A et R₃' sont tels que définis précédemment,
composé de formule (IV) ou (V) qui est soumis à une désalkylation, par exemple qui est soumis à l'action d'un mélange de formule (VI) :

AlHal'₃, R₅-SH (VI)

dans laquelle Hal' représente un halogène et R₅ représente un alkyle linéaire ou ramifié de 1 à 6 atomes de carbone, un aryle ou un aryl-(C₁-C₄)alkyle,
le radical aryle étant par exemple un phényle, pour obtenir un composé de formule (VII) : 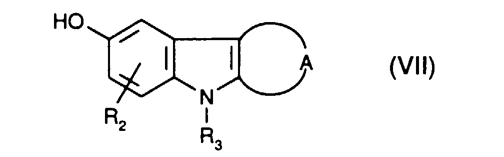 dans laquelle R₂, R₃ et A sont tels que définis précédemment,
composé de formule (VII) qui est mis en réaction avec un composé de formule (VIII) :

Ar-(CH₂)ₙ-Hal'' (VIII)

dans laquelle Ar et n sont tels que définis dans la formule (I) et Hal" représente un halogène,
afin d'obtenir, après une éventuelle saponification pour produire le composé carboxylé à partir de l'ester présent en R₃, le composé de formule (I) : 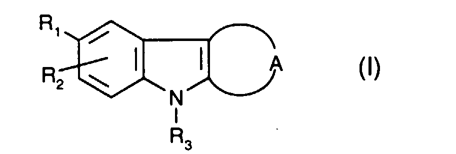 dans laquelle R₁, R₂, R₃ et A sont tels que définis précédemment,
les composés de formule (I) pouvant être, le cas échéant :
- purifiés suivant une ou plusieurs méthodes choisies parmi la cristallisation, la chromatographie sur colonne de silice, l'extraction, la filtration, et le passage sur charbon ou résine,
- séparés, sous forme pure ou sous forme de mélange, en leurs éventuels isomères optiques,
- et transformés, par une base ou un acide, en sels pharmaceutiquement acceptables.

L'invention s'étend également au procédé de préparation des composés de formule (I) caractérisé en ce que l'on condense, en présence d'un amidure de sodium ou sa combinaison avec un alcoolate (base complexe), telle que la base complexe amidure de sodium-tertiobutylate de sodium (NaNH₂-tBuONa), le composé de formule (II') : 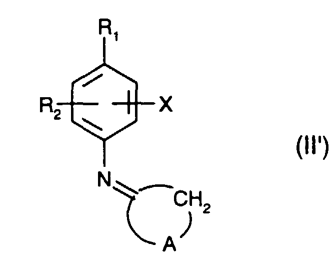 dans laquelle R₁, R₂ et A sont tels que définis dans la formule (I) et X représente un atome d'halogène,
pour obtenir :
- soit un composé de formule (III') : 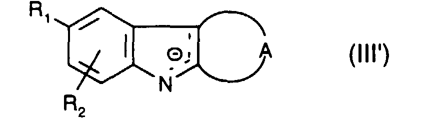 dans laquelle R₁, R₂ et A sont tels que définis précédemment,
- soit, après hydrolyse, un composé de formule (I/a) : 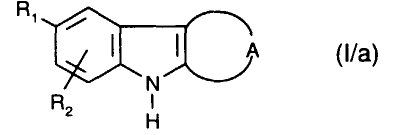 dans laquelle R₁, R₂ et A sont tels que définis précédemment,
composé de formule (III'), ou composé de formule (I/a) sous forme de son sel alcalin, qui est substitué sur l'azote indolique par un radical de formule R₃', dans lequel R_{3'} a la même signification que R₃ tel que défini dans la formule (I) à l'exception de l'hydrogène, pour obtenir un composé de formule (I/b) : 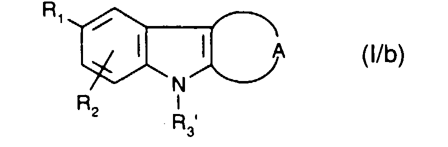 dans laquelle R₁, R₂, A et R₃' sont tels que définis précédemment,
réaction suivie d'une éventuelle saponification pour produire le composé carboxylé à partir de l'ester présent en R₃,
les composés de formule (I/a) et (I/b) formant l'ensemble des composés de formule (I),
les composés de formule (I) pouvant être, le cas échéant :
- purifiés suivant une ou plusieurs méthodes choisies parmi la cristallisation, la chromatographie sur colonne de silice, l'extraction, la filtration, et le passage sur charbon ou résine,
- séparés, sous forme pure ou sous forme de mélange, en leurs éventuels isomères optiques,
- et transformés, par une base ou un acide, en sels pharmaceutiquement acceptables.

La substitution du composé de formule (III) ou (IV), (ou (III') ou (I/a)) tel que définie précédemment par un radical de formule -R₃', prévue dans les procédés de synthèse précédents, est réalisable par réaction d'un composé de formule (III) ou du sel alcalin de formule (IV) (ou (III') ou (I/a) respectivement avec un composé de formule R₃'-X'', Cl-COO-R₃'', (R₃'-O)₂SO₂, X-(CH₂)ₙ-COOR₃'' ou (R₃''-O-CO)₂O dans lesquelles R₃' est tel que défini précédemment, R₃'' représente un radical (C₁-C₆) alkyle et X'' représente un halogène, réaction éventuellement suivie d'une étape d'hydrolyse ou saponification pour l'obtention de la fonction acide à partir de l'ester, lorsque R₃ représente un radical alkoxycarbonylalkyle.

Par exemple l'invention concerne :
- la préparation de la base complexe :
   A une suspension de 7 équivalents (éq.) d'amidure de sodium (dont 2 éq. pour la préparation de l'alcoolate et 1 éq. pour la préparation de l'énolate d'imine ou d'énamine) dans du tétrahydrofurane (7cm³ pour 70 mmoles d'amidure de sodium), on ajoute goutte à goutte 2 éq. de 2-méthylpropan-2-ol à température ambiante. Après cet ajout, le mélange est chauffé à 45°C pendant 2 heures (le rapport NaNH₂/t-BuONa est alors de 2/1 lors de l'étape de cyclisation).
- la cyclisation des imines :
   Au milieu basique préparé ci-dessus, on ajoute, à 0°C, 1 éq. de l'imine à condenser en solution dans le tétrahydrofurane (3 cm³ pour 1 mmole). Le mélange est agité magnétiquement à température ambiante ou 40-45°C. La réaction est suivie par chromatographie en phase gazeuse.
- La préparation des (thia)cycloalkyl[b]indoles non substitués sur l'azote (hydrolyse) :
   Lorsque tout le produit de départ a disparu, on procède à une hydrolyse à 0°C. Après une extraction à l'éther, la phase organique est séchée sur sulfate de magnésium et les solvants évaporés sous pression réduite. Le (thia)cycloalkyl [b]indole est ensuite purifié par chromatographie en phase liquide.
- la N-méthylation *in situ* : préparation des (thia)cycloalkyl[b]indoles N-méthylés :
   Lorsque tout le produit de départ a disparu, le milieu réactionnel est décanté puis la partie liquide est transférée dans une ampoule de Mariotte puis ajoutée goutte à goutte à 3 éq. de sulfate de diméthyle en solution dans du tétrahydrofurane à 0°C. A la fin de l'addition, on laisse le milieu réactionnel remonter à température ambiante. Après 2 heures, le mélange est hydrolysé avec une solution aqueuse d'hydroxyde d'ammonium à 32 % puis extrait au chlorure de méthylène. La phase organique est ensuite lavée à l'eau puis séchée sur sulfate de magnésium et les solvants évaporés sous pression réduite. Le (thia)cycloalkyl[b]indole N-méthylé est ensuite purifié par chromatographie en phase liquide.
- La N-carbéthoxyméthylation *in situ* : préparation des (thia)cycloalkyl[b]indoles N-carbéthoxyméthylés :
   La partie liquide du milieu réactionnel est transférée dans une ampoule à additionner et ajoutée goutte à goutte à une solution de bromoacétate d'éthyle (3 éq.) dans du diméthylformamide (6 cm³ pour 1 mmole, à température ambiante). Après 2 heures, on extrait à l'éther, et la phase organique est lavée à l'eau et séchée sur sulfate de magnésium et les solvants évaporés sous pression réduite. Le thiacycloalkyl[b]indole ainsi obtenu est ensuite purifié par chromatographie en phase liquide.
- la saponification : formation des N-(carboxyméthyl) (thia)cycloalkyl[b]indoles :
   Le N-(carbéthoxyméthyl) (thia)cycloalkyl[b]indole préparé ci-dessus est placé à reflux dans une solution à 10 % d'hydroxyde de potassium dans l'éthanol. La réaction est suivie par chromatographie sur couche mince (ccm).
   Lorsque tout le produit de départ a disparu, la réaction est jetée sur glace et extraite à l'éther. La phase aqueuse est alors acidifiée et extraite à nouveau à l'éther. La phase organique est séchée sur sulfate de magnésium puis les solvants sont évaporés sous pression réduite. Le N-(carboxyméthyl) (thia)cycloalkyl[b]indole est purifié par chromatographie en phase liquide.
- déméthylation : formation des hydroxy(thia)cycloalkyl[b]indoles
   1,5 éq. de chlorure d'aluminium sont mélangés à 20 éq. de phénylméthanethiol à 0°C. Le (thia)cycloalkyl[b]indole (1 éq.) est ajouté en solution dans du chlorure de méthylène (5 cm³ pour 1 mmole). Après une demi-heure de réaction, un mélange identique de chlorure d'aluminium-phénylméthanethiol est ajouté à nouveau au milieu réactionnel. La réaction est suivie par chromatographie couche mince. Lorsque tout le produit de départ a disparu, on procède à une hydrolyse acide et à une extraction au chlorure de méthylène. La phase organique est séchée sur sulfate de sodium puis les solvants évaporés sous pression réduite. L'indole phénolique est isolé par chromatographie en phase liquide.
- formation des 5-benzyloxy(thia)cycloalkyl[b]-indoles
   A 1 éq. de 5-hydroxyindole en solution dans le diméthylformamide (10 cm³ pour 1 mmole), sont ajoutés à température ambiante 1,8 éq. de carbonate de potassium, 3 éq. de phénylméthanethiol et 0,2 éq. de chlorure de triéthylbenzylammonium. Le milieu réactionnel est chauffé à 35-40°C. Lorsque tout le produit de départ a disparu (suivi en ccm), le mélange est jeté sur glace et extrait au dichlorométhane. La phase organique est séchée sur sulfate de magnésium et les solvants évaporés sous pression réduite. Les 5-benzyloxy(thia)cycloalkyl[b]indoles obtenus sont purifiés par chromatographie en phase liquide.
- formation des 5-(2-quinolinoxyméthyl)(thia)cycloalkyl[b]indoles
   Le mode opératoire est identique à celui décrit ci-dessus mais avec 3,6 éq. de carbonate de potassium, 1,5 éq. de chlorhydrate de 2-chlorométhylquinoline et 0,4 éq. de chlorure de triéthylbenzylammonium.

Les composés de formule (II) sont aisément accessibles par réaction d'une aniline de formule (IIa) : 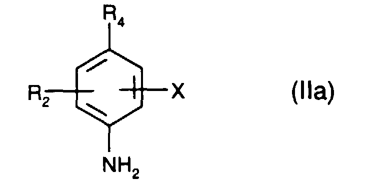 dans laquelle R₂ est tel que défini dans la formule (I), R₄ représente un radical alkoxy linéaire ou ramifié de 1 à 6 atomes de carbone et X représente un halogène,
avec une cétone de formule (IX) : 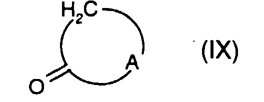 dans laquelle A est tel que décrit dans la formule (I).

Les composés de formule (II') sont aisément accessibles par réaction d'une aniline de formule (IIb) : 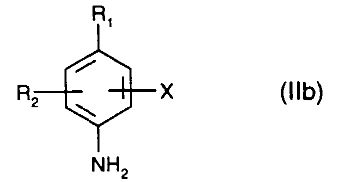 dans laquelle R₁ et R₂ sont tels que définis dans la formule (I), et X représente un halogène,
avec une cétone de formule (IX) 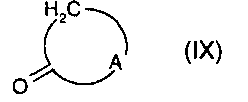 dans laquelle A est tel que décrit dans la formule (I).

Plus particulièrement, les imines peuvent être préparées en soumettant 1 éq. d'halogénoamine et 1 éq. de cétone à une distillation azéotropique dans le benzène. (Selon les cas la réaction peut être catalysée par l'acide para-toluène sulfonique, le chlorure de zinc, le bromure de zinc ou le complexe éther éthylique/trifluorure de bore).
Après avoir récolté la quantité d'eau adéquate, l'imine est distillée sous pression réduite.

Les matières premières utilisées lors des procédés de préparation précédemment décrits sont soit commerciales, soit aisément accessibles à l'homme de l'art d'après la littérature.

De par leur action, les composés de l'invention sont donc de nouveaux candidats cliniques pour le traitement et la prévention des maladies inflammatoires et des conditions inflammatoires pathologiques.

Les composés de l'invention sont de puissants inhibiteurs de la lipoxygénase (Etude pharmacologique : Exemple A) et possèdent une intense activité anti-inflammatoire (Etude pharmacologique: Exemple B).

Les composés de l'invention sont par conséquent utiles dans le traitement et la prévention de l'inflammation chronique ou aigüe, articulaire, pulmonaire, cutanée, ou rénale et notamment dans la prévention et le traitement de l'arthrite, de la polyarthrite rhumatoïde, de l'ostéo-arthrose, du psoriasis, des maladies allergiques, de l'asthme, des maladies inflammatoires de l'intestin, des ulcères gastro-intestinaux, de l'ischémie, de l'athérosclérose, de la détresse respiratoire, et du choc septique.

Les composés de la présente invention possèdent également des propriétés anti-oxydantes très importantes. Les études pharmacologiques ont notamment montré que ces composés sont doués d'activités protectrices remarquables et spécifiques vis-à-vis des peroxydations lipidiques et notamment de peroxydation des lipoprotéines de faible densité (LDL).

La présente invention a également pour objet les compositions pharmaceutiques contenant un des composés de formule (I), ou un de ses sels d'addition à un acide ou à une base, pharmaceutiquement acceptables, seul ou en combinaison avec un ou plusieurs excipients inertes non toxiques.

Parmi les compositions pharmaceutiques selon l'invention, on pourra citer plus particulièrement celles qui conviennent à l'administration orale, percutanée, cutanée, parentérale, nasale, rectale, perlinguale, oculaire ou pulmonaire et notamment les préparations injectables ou buvables, les aérosols, les gouttes oculaires ou nasales, les comprimés simples, pelliculés ou dragéifiés, les gélules, les capsules, les crèmes, les patches, les suppositoires.

La posologie varie selon l'âge, le poids et le sexe du patient, la voie d'administration, la nature et l'intensité de l'affection, et selon les éventuels traitements associés. Les doses s'échelonnent entre 0,5 mg à 2 g par jour, particulièrement entre 0,5 mg à 100 mg par jour, par exemple entre 5 mg à 50 mg par jour.

### Préparation 1: N-cyclo-octylidényl-3-chloro-4-méthoxyaniline

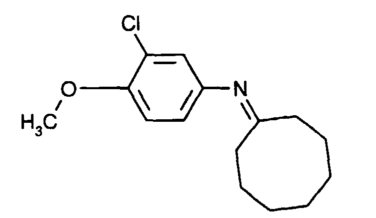

1 éq. de 3-chloropara-anisidine et 1 éq. de cyclo-octanone sont soumis à une distillation azéotropique dans du toluène (100 cm³ pour 50 mmoles). La réaction est suivie par chromatographie gazeuse et arrêtée au bout de 48 h. L'imine est purifiée par distillation et est obtenue sous forme de liquide avec un rendement de 68 %.
RMN ¹ H, δ (ppm) : 6,2-6,9 (3H, m, H arom.) ; 3,8 (3H, s, MeO ); 1,3-2,8 (14H, m, 7 x CH₂)
I.R. : 2930-2857 (C-H) ; 1644 (N=C)

### Préparation 2: N-cycloheptylidényl-3-chloro-4-méthoxyaniline

Le composé du titre est obtenu par distillation azéotropique à partir de la 3-chloro-4-méthoxyaniline et de la cycloheptanone, en utilisant l'acide paratoluènesulfonique comme catalyseur et le benzène comme solvant.
La réaction est suivie par chromatographie en phase gazeuse. Après achèvement de la réaction, le milieu est ramené à température ambiante, traité par une solution saturée en hydrogénocarbonate de sodium, extrait par de l'éther et séché sur sulfate de magnésium. Les solvants sont éliminés sous vide. L'imine est purifiée par distillation ou utilisée telle quelle.

### Préparation 3 : N-(thiopyranilidén-3-yl)-3-chloro-4-méthoxyaniline

1 éq. de 3-chloropara-anisidine et 1 éq. de thiopyran-3-one sont soumis à une distillation azéotropique dans du benzène (100 cm³ pour 50 mmoles). La réaction est suivie par chromatographie gazeuse et arrêtée au bout de 5 h. L'imine est purifiée par distillation.
Rendement : 40%
RMN ¹H, δ (ppm) : 6,3-7,0 (3H, m, H arom.) ; 3,9 (3H, s, OMe) ; 3,3 (1H, s, C-H) ; 3,1 (1H, s, C-H) ; 2,0-3,0 (m, 6H, 3 x CH₂)

### EXEMPLE 1: 2-(3-BENZYLOXY-5,6,7,8,9,10,11-HEPTAHYDROCYCLO-OCT[b] INDOL-11-YL)ACETATE D'ETHYLE

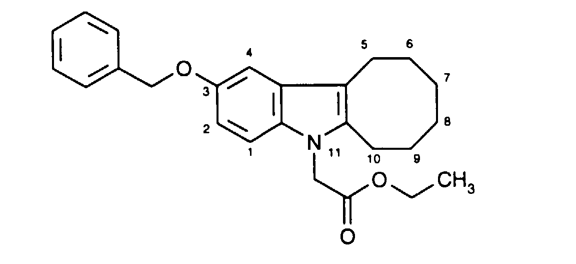

### STADE A : 3-méthoxy-5,6,7,8,9,10,11-heptahydrocyclo-oct[b]indole

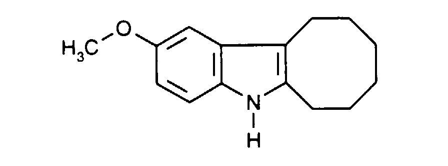

### Préparation de la base complexe :

A une suspension de 7 équivalents d'amidure de sodium dans du tétrahydrofurane (7 cm³ pour 70 mmoles d'amidure), on ajoute goutte à goutte 2 éq. de 2-méthylpropan-2-ol à température ambiante. Après cet ajout, le mélange est chauffé à 45°C pendant 2 heures.

### Condensation :

A la base complexe préparée ci-dessus, on ajoute, à 0°C, 1 éq. de N-cyclooctylidényl-3-chloro-4-méthoxyaniline(préparation 1) dans du tétrahydrofurane (30 cm³ pour 10 mmoles). Le mélange est agité à température ambiante pendant 24 h. La réaction est suivie par chromatographie gazeuse. A la fin de la réaction, le mélange est jeté sur glace, extrait à l'éther. Après séchage sur sulfate de magnésium et évaporation des solvants sous pression réduite, l'indole est isolé par chromatographie éclair (éluant: acétate d'éthyle/éther de pétrole, 10 : 90).
Rendement : 77 %
Point de fusion : 102°C
I.R. : 3405 (NH) ; 2922, 2848 (C-H)

| Analyse élémentaire (C₁₅H₁₉ON poids moléculaire : 229,32) : | | | |
|---|---|---|---|
| | **C** | **H** | **N** |
| **% calculé** | 78,56 | 8,35 | 6,10 |
| **% trouvé** | 78,65 | 8,43 | 6,10 |

### STADE B : 2-(3-méthoxy-5,6,7,8,9,10,11-heptahydrocyclo-oct[b]indol-11-yl) acétate d'éthyle

### Procédure A :

Le composé synthétisé au stade A est additionné à 0°C en solution, dans du diméthylformamide (5 cm³ pour 1 mmole), à 1,2 éq. d'hydrure de sodium.
Lorsque l'addition est complète, le milieu réactionnel est agité à température ambiante pendant 5 minutes. On ajoute alors, goutte à goutte, 2 éq. de bromoacétate d'éthyle et la réaction est agitée à température ambiante. La réaction est suivie en chromatographie sur couche mince (ccm). Lorsque la réaction est terminée, le milieu réactionnel est jeté sur glace, extrait au chlorure de méthylène. Après séchage sur sulfate de magnésium et évaporation des solvants sous pression réduite, le composé du titre est isolé par chromatographie éclair (éluant : acétone/hexane, 10: 90)
Rendement : 72 %
Point de fusion : 50°C
I.R. : 2982, 2928, 2850 (C-H) ; 1753 (C=O)

| Analyse élémentaire (C₁₉H₂₅O₃ N poids moléculaire : 315,41) : | | | |
|---|---|---|---|
| | **C** | **H** | **N** |
| **% calculé** | 72,35 | 7,98 | 4,44 |
| **% trouvé** | 71,78 | 8,10 | 4,36 |

### Procédure B :

A la fin de la condensation effectuée au stade A, le milieu réactionnel est laissé décanté sous courant d'azote. Le surnageant est transféré, à température ambiante, dans une solution de 4 éq. de bromoacétate d'éthyle dans le diméthylformamide (de façon à avoir un mélange tétrahydrofurane/diméthylformamide = 1/2 à la fin de l'addition). Lorque la réaction est terminée (suivie en ccm), on procède comme ci-dessus (Procédure A).
Rendement : 42 %

### STADE C : 2-(3-hydroxy-5,6,7,8,9,10,11-heptahydro-cyclo-oct[b]indol-11-yl) acétate d'éthyle

A une suspension de 1,5 éq. de chlorure d'aluminium et 20 éq. de phénylméthanethiol à 0°C, on ajoute 1 éq. du composé obtenu au stade B en solution dans du chlorure de méthylène sec (5 cm³ pour 1 mmole). La réaction est agitée magnétiquement pendant 1/2 h avant d'ajouter à nouveau 1,5 éq. de chlorure d'aluminium et 20 éq. de phénylméthanethiol. Le milieu réactionnel est encore agité 1 h 30 à 0°C. Après hydrolyse acide à 0°C (acide chlorhydrique 1N), on procède à une extration avec du chlorure de méthylène, puis un lavage à l'eau et une solution saturée en chlorure de sodium. Après séchage sur sulfate de sodium et évaporation des solvants, le 2-(3-hydroxy-5,6,7,8,9,10,11-heptahydro-cyclooct[b]indol-11-yl) acétate d'éthyle est isolé par chromatographie éclair (éluant : acétate d'éthyle/éther de pétrole, 20 : 80).
Rendement : 60 %
Point de fusion : 100°C
I.R. : 3406 (OH) ; 2980, 2927, 2850 (C-H) ; 1753 (C=O)

| Analyse élémentaire (C₁₈H₂₃O₃N poids moléculaire : 301,39) : | | | |
|---|---|---|---|
| | **C** | **H** | **N** |
| **% calculé** | 71,73 | 7,69 | 4,64 |
| **% trouvé** | 71,49 | 7,67 | 4,77 |

### STADE D : 2-(3-benzyloxy-5,6,7,8,9,10,11-heptahydrocylclo-oct[b]indol-11-yl) acétate d'éthyle

A une solution du composé obtenu au stade C dans le diméthylformamide (10 cm³ pour 1 mmole), on ajoute, à température ambiante, 1,8 éq. de carbonate de potassium puis 1,2 éq. de chlorure de benzyle et enfin 0,2 éq. de chlorure de triéthylbenzylammonium. La réaction est chauffée à 35-40°C pendant 5 h et suivie par ccm. Lorsque la réaction est terminée, on procède à une hydrolyse sur glace puis à une extraction avec du chlorure de méthylène. Après lavage à l'eau, séchage sur sulfate de magnésium et évaporation des solvants sous pression réduite, le composé du titre est isolé par chromatographie éclair (éluant : acétate d'éthyle/éther de pétrole, 15 : 85).
Rendement : 77 %
Point de fusion : huile
I.R. : 2927, 2850 (C-H) ; 1753 (C=O)

| Analyse élémentaire (C₂₅H₂₉O₃N poids moléculaire : 391,51) : | | | |
|---|---|---|---|
| | **C** | **H** | **N** |
| **% calculé** | 76,69 | 7,46 | 3,57 |
| **% trouvé** | 76,76 | 7,51 | 3,61 |

### EXEMPLE 2: 2-{3-[(QUINOL-2-YL)METHYLOXY]-5,6,7,8,9,10,11-HEPTAHYDROCYCLO-OCT[b]INDOL-11-YL}ACETATE D'ETHYLE

A une solution du composé obtenu au stade C de l'exemple 1 dans le diméthylformamide (10cm³ pour 1 mmole), on ajoute, à température ambiante, 1,8 éq. (x2) de carbonate de potassium, 1,5 éq. de 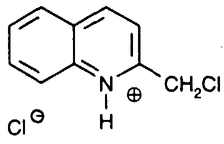 et enfin 0,2 eq. (x2) de chlorure de triéthylbenzylammonium. La réaction est chauffée à 40°C pendant 20 h et suivie par ccm. Lorsque la réaction est terminée, on procède à une hydrolyse sur glace puis à une extraction avec du chlorure de méthylène. Après lavage à l'eau, séchage sur sulfate de magnésium et évaporation des solvants sous pression réduite, le composé du titre est isolé par chromatographie éclair (éluant : acétate d'éthyle/éther de pétrole, 15 : 85).
Rendement : 86 %
Point de fusion : 60°C
I.R. : 2925, 2852 (C-H) ; 1753 (C=O)

### EXEMPLE 3: ACIDE 2-(3-BENZYLOXY-5,6,7,8,9,10,11-HEPTAHYDROCYCLO-OCT[b]INDOL-11-YL)ACETIQUE

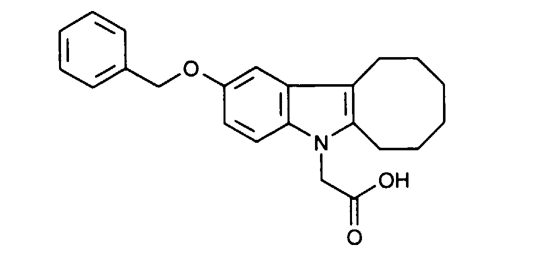

Le composé obtenu à l'exemple 1 (stade D) est placé dans une solution d'hydroxyde de potassium dans l'éthanol à 10 % et porté à reflux pendant 3 h. La solution est extraite à l'éther, puis la phase aqueuse est acidifiée et extraite à nouveau à l'éther. Après lavage à l'eau, séchage sur sulfate de magnésium et évaporation des solvants, le composé du titre est isolé par chromatographie éclair (éluant : acétate d'éthyle/éther de pétrole, 50 : 50).
Rendement : 65 %
Point de fusion : 105°C
I.R. : 3700 à 2200 (OH) ; 2923, 2849 (C-H) ; 1720 (C=O)

| Analyse élémentaire (C₂₃H₂₅O₃N poids moléculaire : 399,49) : | | | |
|---|---|---|---|
| | **C** | **H** | **N** |
| **% calculé** | 76,00 | 6,93 | 3,85 |
| **% trouvé** | 75,99 | 7,23 | 3,94 |

I.R. : 3600 à 2200 (OH) ; 1750 (C=O)
Point de fusion (chlorhydrate) : 135°C

### EXEMPLE 4: ACIDE 2-{3-[(QUINOL-2-YL)METHYLOXY]-5,6,7,8,9,10,11-HEPTAHYDROCYCLO-OCT[b]INDOLE}ACETIQUE

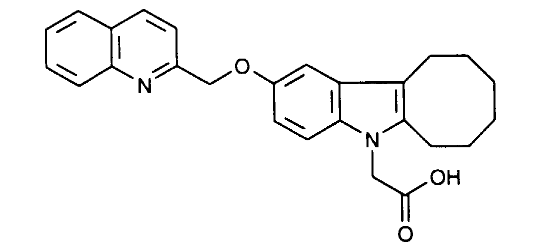

En procédant comme dans l'exemple 3, mais en partant du composé de l'exemple 2, on obtient le composé du titre.
Point de fusion (chlorhydrate): 135°C
I.R. : 3600 à 2200 (OH) ; 1750 (C=O)

### EXEMPLE 5: 2-(3-BENZYLOXY-5,6,7,8,9,10-HEXAHYDROCYCLOHEPT[b]INDOL-10-YL)ACETATE D'ETHYLE

### STADE A : 3-méthoxy-5,6,7,8,9,10-hexahydrocyclohept[b]indole

### Préparation de la base complexe:

A une suspension de 7 équivalents d'amidure de sodium dans du tétrahydrofurane (7 cm³ pour 70 mmoles d'amidure de sodium), on ajoute goutte à goutte 2 éq. de 2-méthylpropan-2-ol à température ambiante. Après cet ajout, le mélange est chauffé à 45°C pendant 2 heures.

### Condensation :

On ajoute, à 0°C, 1 eq. de N-cyclohepthylidényl-3-chloro-4-méthoxyaniline (préparation 2) à la base complexe préparée ci-dessus. Le mélange est agité à température ambiante jusqu'à achèvement de la réaction. La réaction est suivie par chromatographie gazeuse. Le 3-méthoxy-5,6,7,8,9,10-hexahydrocyclohept[b]indole est séparé par chromatographie en phase liquide.

Puis, procédant comme dans les stades B à D de l'exemple 1 mais en partant du composé obtenu au stade A, on obtient les composés des stades suivants :
- STADE B :: 2-(3-méthoxy-5,6,7,8,9,10-hexahydrocyclohept[b]indol-10-yl)acétate d'éthyle
- STADE C :: 2-(3-hydroxy-5,6,7,8,9,10-hexahydrocyclohept[b]indol-10-yl)acétate d'éthyle
- STADE D :: 2-(3-benzyloxy-5,6,7,8,9,10-hexahydrocyclohept[b]indol-10-yl)acétate d'éthyle

### EXEMPLE 6: 2-{3-[(QUINOL-2-YL)METHYLOXY]-5,6,7,8,9,10-HEXAHYDROCYCLOHEPT[b]INDOL-10-YL}ACETATE D'ETHYLE

Point de fusion : 66°C

### EXEMPLE 7 : ACIDE 2-(3-BENZYLOXY-5,6,7,8,9,10-HEXAHYDROCYCLOHEPT[b]INDOL-10-YL)ACETIQUE

### EXEMPLE 8: ACIDE 2-{3-[(QUINOL-2-YL)METHYLOXY]-5,6,7,8,9,10-HEXAHYDROCYCLOHEPT[b]INDOL-10-YL}ACETIQUE

### EXEMPLE 9: 2-{8-[(QUINOL-2-YL)METHYLOXY]THIOPYRANO[3,2-b]INDOL-5-YL}ACETATE D'ETHYLE

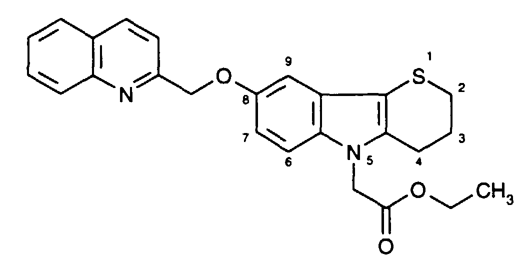

### METHODE A

### STADE A : 2-(8-hydroxythiopyrano[3,2-b]indol-5-yl)acétate d'éthyle

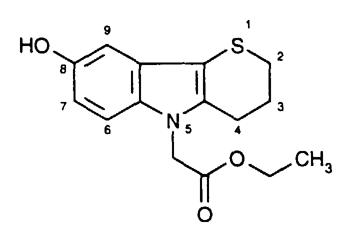

Dans du toluène à reflux (100 ml pour 50 mmol) sont placés 1 éq. de 3-chloro-4-(tétrahydropyran-2-yloxy)phénylamine et 1 éq. de dihydrothiopyran-3-one en présence de 0,035 éq. d'acide acétique. La réaction est suivie par chromatographie en phase gazeuse. Au bout de 31 heures, on procède à une neutralisation avec du carbonate de potassium. Après filtration, séchage sur sulfate de magnésium, le solvant est évaporé sous pression réduite. L'imine ainsi obtenue, en solution dans le tétrahydrofurane (30 ml pour 10 mmol) est ajoutée à 0°C dans la base complexe amidure de sodium/tertiobutylate de sodium (5 : 2). La réaction est suivie par chromatographie en phase gazeuse. A la fin de la condensation (après 24 heures), le milieu réactionnel est laissé décanté sous azote. Le surnageant est transféré, à température ambiante, dans une solution de 4 éq. de bromoacétate d'éthyle dans le diméthylformamide (de façon à avoir un mélange tétrahydrofurane/diméthylformamide de 1/2 à la fin de l'addition). Lorsque la réaction est terminée (suivie par chromatographie sur couche mince), on procède à une hydrolyse à 0°C en jetant le milieu réactionnel sur glace. On extrait alors plusieurs fois à l'éher. La phase organique est séchée sur sulfate de magnésium, puis les solvants sont évaporés sous pression réduite. Le brut réactionnel obtenu est placé dans du méthanol (1 ml pour 1 mmol) en présence de 0,2 éq. d'acide paratoluènesulfonique. Lorsque la réaction est terminée (suivie par chromatographie sur couche mince), le méthanol est évaporé. Le résidu obtenu est dilué dans l'éther, lavé avec une solution d'hydrogénocarbonate de sodium à 5%. La phase organique est séchée sur sulfate de magnésium puis le solvant évaporé sous pression réduite. Le produit brut obtenu correspondant au composé attendu est purifié par chromatographie éclair (éluant : acétone/hexane, 20 : 80).
Rendement : 25%
I.R. : 3449 (OH) ; 1741 (C=O)
RMN ¹H, δ (ppm) : 7,00-6,70 (m, 3H, H_{arom.}) ; 4,80 (s, 1H, OH) ; 4,70 (s, 2H, CH₂CO₂Et) ; 4,15 (q, 2H, CO₂CH₂CH₃) ; 3,00-2,20 (m, 6H, 3 × CH₂) ; 1,20 (t, 3H, CO₂CH₂CH₃).

### STADE B : 2-{8-[(quinol-2-yl)méthyloxy]thiopyrano[3,2-b]indol-5-yl}acétate d'éthyle

A une solution de 1 éq. du composé obtenu au stade A dans le diméthylformamide (10 ml pour 1 mmol), on ajoute, à température ambiante, 2 éq. (x2) de carbonate de potassium puis 1,5 éq. de chlorhydrate de 2-chlorométhylquinoléine et enfin 0,2 éq. (x2) d'hydrogénosulfate de tétrabutylammonium. Le milieu réactionnel est chauffé à 40°C pendant 27 heures. La réaction est suivie par chromatographie sur couche mince. Lorsque le produit de départ a disparu, on procède à une hydrolyse sur glace puis à une extraction à l'éther. Après lavage à l'eau puis séchage sur sulfate de magnésium, et évaporation des solvants sous pression réduite, le 2-{8-[(quinol-2-yl)méthyloxy]thiopyrano[3,2-b]indol-5-yl}acétate d'éthyle est purifié par chromatographie éclair (éluant: méthanol/dichlorométhane, 2 : 998).
Rendement : 50%

### METHODE B

### STADE A : 8-methoxy-thiopyrano[3,2-b]indole

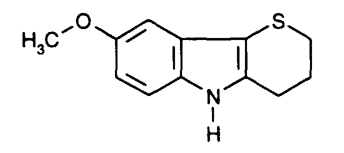

### Préparation de la base complexe :

A une suspension de 7 éq. d'amidure de sodium dans du tétrahydrofurane (7 cm³ pour 70 mmoles d'amidure de sodium), on ajoute goutte à goutte 2 éq. de 2-méthylpropan-2-ol à température ambiante. Après cet ajout, le mélange est chauffé à 45°C pendant 2 heures.

### Condensation :

### Procédure 1 :

A la base complexe préparée ci-dessus, on ajoute à 0°C, 1 éq. de N-(thiopyranylidén-3-yl)-3-chloro-4-méthoxy-aniline (préparation 3) en solution dans du tétrahydrofurane (30 cm³ pour 10 mmoles).
Le mélange est agité à température ambiante pendant 12 h. La réaction est suivie par chromatographie gazeuse. A la fin de la réaction, le milieu réactionnel est jeté sur glace et extrait à l'éther.
Après séchage sur sulfate de magnésium et évaporation des solvants sous pression réduite, le composé du titre est isolé par chromatographie éclair (Kieselgel 40-63 Mesh) (éluant: acétate d'éthyle/éther de pétrole, 5 : 95).
Rendement : 53 %
Point de fusion: 123°C
I.R. : 3390 (NH), 2999, 2938, 2922, 2834 (C-H)

| Analyse élémentaire (C₁₂H₁₃ONS poids moléculaire : 219,30) : | | | | |
|---|---|---|---|---|
| | **C** | **H** | **N** | **S** |
| **% calculé** | 65,72 | 5,97 | 6,38 | 14,62 |
| **% trouvé** | 65,95 | 6,03 | 6,53 | 14,93 |

### Procédure 2 :

L'imine brute obtenue dans la préparation 3 à partir de 1 éq. de cétone et de 1 éq. d'amine, est ajoutée à 0°C, sans purification, à la base complexe préparée ci-dessus. La réaction est effectuée comme pour la procédure 1.
Rendement : 36, 5 % par rapport à la cétone.

### STADE B : 2-(8-méthoxy-thiopyrano[3,2-b]indol-5-yl) acétate d'éthyle

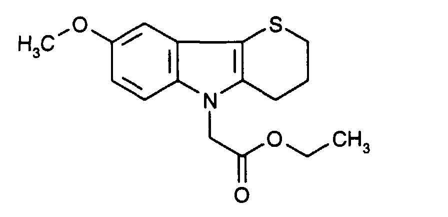

### Procédure 1 :

Le milieu réactionnel brut obtenu à l'étape précédente est laissé décanté sous courant d'azote. Le surnageant obtenu est transferé dans une solution de 4 éq. de bromoacétate d'éthyle dans le diméthylformamide (de façon à avoir un mélange THF/DMF = 1/2 à la fin de l'addition), à température ambiante.
Lorsque la réaction est terminée, le milieu réactionnel est jeté sur glace puis extrait à l'éther. Le traitement habituel de la phase organique fournit un composé brut qui est purifié par chromatographie éclair (éluant : acétone/hexane, 15 : 85).
Rendement : 46 %
Point de fusion: 98°C
I.R. : 2923 (CH), 1750 (C=O)

| Analyse élémentaire (C₁₆H₁₃O₃NS poids moléculaire : 299,34) : | | | | |
|---|---|---|---|---|
| | **C** | **H** | **N** | **S** |
| **% calculé** | 62,92 | 6,27 | 4,58 | 10,49 |
| **% trouvé** | 62,58 | 6,32 | 4,69 | 10,36 |

### Procédure 2 :

L'indole obtenue au stade A peut être également additionnée, à 0°C en solution dans le diméthylformamide (5 cm³ pour 1 mmol) à 1,2 éq. d'hydrure de sodium. Lorsque que l'addition est complète, le milieu réactionnel est agité à température ambiante pendant 5 minutes. On ajoute alors, goutte à goutte, 2 éq. de bromoacétate d'éthyle et le milieu réactionnel est agité à température ambiante. Lorsque la réaction est terminée, le milieu réactionnel est jeté sur glace puis extrait à l'éther. Le traitement habituel de la phase organique fournit un composé brut qui est purifié par chromatographie éclair (éluant: acétone/hexane, 15 : 85).

### STADE C : 2-(8-hydroxy-thiopyrano[3,2-b]indol-5-yl)acétate d'éthyle

En procédant comme pour le stade C de l'exemple 1, on obtient le composé du titre.

### STADE D : 2-{8-[(quinol-2-yl)méthyloxy]thiopyrano[3,2-b]indol-5-yl}acétate d'éthyle

En procédant comme pour l'exemple 2, on obtient le composé du titre.

### EXEMPLE 10: ACIDE 2-{8-[(QUINOL-2-YL)METHYLOXY]THIOPYRANO[3,2-b]INDOL-5-YL}ACETIQUE

Le composé obtenu à l'exemple 9 est placé dans une solution d'hydroxyde de potassium à 10 % dans l'éthanol et porté à reflux pendant 3 heures. Le mileu réactionnel est extrait à l'éther, puis la phase aqueuse est acidifiée et extraite à nouveau à l'éther. Après traitement habituel de la phase organique, le composé brut attendu est purifié par recristallisation dans le diméthylformamide.
Point de fusion: 215°C
I.R. : 3463 (OH), 1724 (C=O)

| Analyse élémentaire (C₂₃H₂₀O₃N₂S poids moléculaire : 404,48) : | | | | |
|---|---|---|---|---|
| | **C** | **H** | **N** | **S** |
| **% calculé** | 68,30 | 4,98 | 6,92 | 7,93 |
| **% trouvé** | 68,42 | 5,07 | 6,85 | 7,67 |

### EXEMPLES 11 à 26 :

En utilisant les méthodes décrites précédemment mais en partant des réactifs appropriés, on obtient les composés des exemples suivants, représentatifs de l'invention :

### EXEMPLE 11: 2-{3-[(THIAZOL-2-YL)METHYLOXY]-5,6,7,8,9,10,11-HEPTA-HYDROCYCLO-OCT[b]INDOL-11-YL}ACETATE D'ETHYLE

### EXEMPLE 12 : ACIDE 2-{3-[(THIAZOL-2-YL)METHYLOXY]-5,6,7,8,9,10,11-HEPTA-HYDROCYCLO-OCT[b]INDOL-11-YL}ACETIQUE

### EXEMPLE 13: 2-{3-{[4-(PROP-2-YL)THIAZOL-2-YL]METHYLOXY}-5,6,7,8,9,10,11-HEPTAHYDROCYCLO-OCT[b]INDOL-11-YL}ACETATE D'ETHYLE

### EXEMPLE 14 : 2-{3-[(5-CHLOROOXAZOL-2-YL)METHYLOXY)-5,6,7,8,9,10,11-HEPTAHYDROCYCLO-OCT[b]INDOL-11-YL}ACETATE D'ETHYLE

### EXEMPLE 15 : 2-{3-[(1,2,5-THIADIAZOL-3-YL)METHYLOXY)-5,6,7,8,9,10,11-HEPTAHYDROCYCLO-OCT[b]INDOL-11-YL}ACETATE D'ETHYLE

### EXEMPLE 16 : 2-{3-[(THIAZOL-4-YL)METHYLOXY)-5,6,7,8,9,10,11-HEPTA-HYDROCYCLO-OCT[b]INDOL-11-YL}ACETATE D'ETHYLE

### EXEMPLE 17 : ACIDE 2-{3-[(THIAZOL-4-YL)METHYLOXY]-5,6,7,8,9,10,11-HEPTA-HYDROCYCLO-OCT[b]INDOL-11-YL}ACETIQUE

### EXEMPLE 18 : 2-{3-[(THIEN-2-YL)METHYLOXY]-5,6,7,8,9,10,11-HEPTAHYDRO-CYCLO-OCT[b]INDOL-11-YL}ACETATE D'ETHYLE

### EXEMPLE 19 : 2-{3-[(2-METHYLTHIAZOL-4-YL)METHYLOXY]-5,6,7,8,9,10,11-HEPTAHYDROCYCLO-OCT[b]INDOL-11-YL}ACETATE D'ETHYLE

### EXEMPLE 20 : ACIDE 2-{3-[(2-METHYLTHIAZOL-4-YL)METHYLOXY]-5,6,7,8,9,10,11-HEPTAHYDROCYCLO-OCT[b]INDOL-11-YL}ACETIQUE

### EXEMPLE 21 : 2-{3-[(6-CHLOROPYRID-2-YL)METHYLOXY]-5,6,7,8,9,10,11-HEPTAHYDROCYCLO-OCT[b]INDOL-11-YL}ACETATE D'ETHYLE

### EXEMPLE 22 : 2-{3-[(PYRID-3-YL)METHYLOXY]5,6,7,8,9,10,11-HEPTAHYDRO-CYCLO-OCT[b]INDOL-11-YL}ACETATE D'ETHYLE

### EXEMPLE 23 : 2-{3-[(NAPHT-2-YL)METHYLOXY]-5,6,7,8,9,10,11-HEPTAHYDRO-CYCLO-OCT[b]INDOL-11-YL}ACETATE D'ETHYLE

### EXEMPLE 24 : 2-{3-[(4,6-DIMETHYLPYRID-2-YL)METHYLOXY]-5,6,7,8,9,10,11-HEPTAHYDROCYCLO-OCT[b]INDOL-11-YL}ACETATE D'ETHYLE

### EXEMPLE 25 : 2-{3-[(THIAZOL-2-YL)METHYLOXY]-5,6,7,8,9,10-HEXAHYDROCYCLO-HEPT[b]INDOL-10-YL}ACETATE D'ETHYLE

### EXEMPLE 26 : 2-{3-[(4,6-DIMETHYLPYRID-2-YL)METHYLOXY]-5,6,7,8,9,10-HEXAHYDROCYCLO-HEPT[b]INDOL-10-YL}ACETATE D'ETHYLE

### ETUDE PHARMACOLOGIQUE

### EXEMPLE A : RECHERCHE D'UNE INTERACTION DES COMPOSES DE L'INVENTION AVEC LES ACTIVITES LIPOXYGENASE ET CYCLO-OXYGENASE DE GRANULOCYTES DE LAPIN

### But de l'étude :

L'objet de cette étude est de rechercher les effets des composés de l'invention sur les activités lipoxygénase et cyclooxygénase de granulocytes de lapin stimulés *in vitro* par le A 23187, en mesurant les taux de PGE₂ et de LTB₄ ainsi libérés par "Enzymo Immuno Assay" (EIA).

### Matériels et méthodes :

- *Obtention des granulocytes*
Les granulocytes sont isolés à partir de sang de lapin recueilli à l'artère principale de l'oreille selon la méthode décrite par Trush et Coll. (1978).
Ils sont suspendus, à une densité de 10⁶ cellules/cm³ dans du tampon phosphate (mM) : NaCl : 137 ; KCI : 2,68 ; Na₂HPO₄ : 8,1 ; KH₂PO₄ : 1,47 ; CaCl₂ : 0,9 ; MgCl₂ : 0,5
- *Stimulation des cellules*
Les granulocytes fraîchement obtenus sont préincubés pendant 15 min à 37°C en présence des produits à tester.
- *Produits utilisés*

| | |
|---|---|
| Kit EIALTB₄ | Amersham ® |
| Kit TIA PGE₂ | Amersham ® |
| A 23187 | Sigma® |
| NDGA | Sigma® |
| Indométhacine | Sigma® |
| Produits courants | Sigma® |

- *Analyse des résultats*
Les résultats sont exprimés en pourcentages du témoin positif. Les valeurs sont présentées sous forme de moyenne ± e.s.m. de 3 mesures effectuées le même jour, pour l'étude "screening" et de 3 mesures effectuées sur trois jours différents, pour l'étude IC₅₀.
Les valeurs des IC₅₀ et des nombres de Hill (nH) sont déterminées par modélisation de l'équation de Hill.

### Résultats :

Les composés de l'invention se révèlent être de puissants inhibiteurs de la voie de la 5-lipoxygénase. Cette action est sélective et n'interfère pas avec la voie de la cyclooxygénase.
Ainsi à 10⁻⁷ M, les composés des exemples 2, 3 et 6 inhibent totalement la production de LTB₄ sans dimininuer celle de PGE₂.

### EXEMPLE B : ETUDE DE L'ACTIVITE ANTI-INFLAMMATOIRE : ETUDE IN VIVO

Le potentiel anti-inflammatoire des composés a été recherché sur un modèle d'inflammation aigüe provoquée par injection sous cutanée d'une suspension colloïdale de carragénine au niveau de la face plantaire de la patte postérieure de rat, selon une technique inspirée de la méthode de WINTER, RISLEY et NUSS, *Proc. Soc. Exp. Biol. Med., 111,* 554, (1962), et WINEGAR et coll., *J. Pharmacol. Exp. Ther., 166,* 96, (1969).

Les rats mâles WISTAR EOPS sont "randomisés" par lot de 10 et reçoivent les substances à tester *per os* 1 heure après l'injection au niveau de la patte arrière gauche de 0,15 cm³ d'une suspension à 1% de carraghénate. L'inflammation est mesurée 5 heures plus tard par pesée des pattes et les pourcentages d'inflammation et d'activité anti-inflammatoire (AAI) sont calculés.

### Résultats :

Il apparaît que les composés de l'invention administrés par voie orale à la dose de 10 mg/kg permettent une réduction très importante de volume de l'oedème provoqué.

### EXEMPLE C: ETUDE DE LA TOXICITE AIGUE

La toxicité aigüe a été appréciée après administration orale à des lots de 3 souris (20 ± 2 grammes) de doses croissantes (0,1 - 0,25 - 0,50 - 0,75 - 1g/kg) de composés de l'invention. Les animaux ont été observés à intervalles réguliers au cours de la première journée et quotidiennement pendant les 2 semaines suivant le traitement. Il apparaît que les composés de l'invention sont totalement atoxiques. Aucun décès n'est observé après une administration d'une dose de 1 g/kg. On ne constate pas de trouble après administration de cette dose.

### EXEMPLE D : Etude des composés de l'invention dans le modèle d'inflammation cutanée aigüe chez la souris (Test au 12-myristate-13-acétate de phorbol)

Dans le test suivant, les mesures ont été effectuées sur des groupes de 5 souris. Les tests sur les groupes de souris témoin sont effectués simultanément avec les groupes de souris ayant reçu les composés à tester.

5 µg de 12-myristate-13-acétate de phorbol dans 20 µl d'un mélange éthanol/eau (8:2) sont appliqués sur les faces antérieure et postérieure de l'oreille droite de la souris, 30 minutes après l'application similaire des composés à tester ou du véhicule (2 x 20 µl à intervalles de 5 minutes dans l'alcool absolu). Le gonflement de l'oreille est mesuré à l'aide d'un micromètre type Dyer après 6 heures. Cette mesure est utilisée comme index d'inflammation.

Les résultats obtenus sont présentés dans le tableau suivant :

| ***Composé*** | ***IC***_{***50***} ***(µM)*** |
|---|---|
| Exemple 2 | 20 |
| Exemple 3 | 170 |
| Exemple 4 | 15 |
| Exemple 6 | 10 |

### EXEMPLE E: COMPOSITION PHARMACEUTIQUE : COMPRIMES

Formule de préparation pour 1000 comprimés dosés à 50 mg.

| | |
|---|---|
| 2-{3-[(quinol-2-yl)méthoxy]-5,6,7,8,9,10,11-heptahydrocyclooct[b]indol-11-yl}acétate d'éthyle | 50 g |
| Amidon de blé | 15 g |
| Amidon de maïs | 15 g |
| Lactose | 65 g |
| Stéarate de magnésium | 2 g |
| Silice | 1 g |
| Hydroxypropylcellulose | 2 g |

## Revendications

1. Composés de formule (I) : 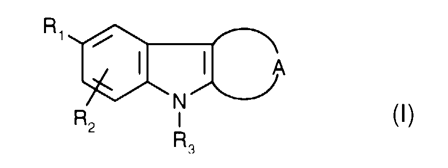 dans laquelle :
- R₁ représente un groupement Ar-(CH₂)ₙ-O- avec n représentant zéro ou un entier de 1 à 4, Ar étant un radical choisi parmi phényle, naphtyle, thiényle, furyle, pyrrolyle, imidazolyle, pyrazolyle, thiazolyle, isothiazolyle, oxazolyle, isoxazolyle, thiadiazole, oxadiazole, pyridyle, quinolyle, isoquinolyle, indolyle, benzofuryle, et benzothiényle ; Ar pouvant être non substitué ou substitué par un ou plusieurs radicaux choisis parmi halogène, alkyle, alkoxy, hydroxy et trifluorométhyle,
- R₂ représente un atome d'hydrogène ou un radical choisi parmi halogène, alkyle, alkoxy et trifluorométhyle,
- R₃ représente un radical choisi parmi hydrogène, alkyle, carboxyalkyle et alkoxycarbonylalkyle,
- A représente une chaîne alkylène -(CH₂)ₘ- avec m compris entre 3 et 6 ou un groupement de formule (α) : 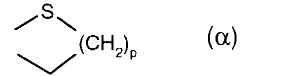 dans lequel p est compris entre 1 et 4,
étant entendu que les termes "alkyle" et "alkoxy" désignent des groupements linéaires ou ramifiés de 1 à 6 atomes de carbone,
étant entendu que le composé de formule (I) ne peut représenter le 7-benzyloxy-1,2,3,4-tétrahydrocyclopenta[b]indole, le 7-benzyloxy-4*H*-2,3-dihydrothiéno[3,2-b]indole, le 6-benzyloxy-1,2,3,4-tétrahydrocarbazole, ni le 6-benzyloxy-9-méthyl-1,2,3,4-tétrahydrocarbazole,
leurs isomères optiques, sous forme pure ou sous forme de mélange,
et leurs sels d'addition à un acide ou une base, pharmaceutiquement acceptables.

2. Composés selon la revendication 1 dans lesquels A représente une chaîne pentaméthylène,
leurs isomères optiques, sous forme pure ou sous forme de mélange,
et leurs sels d'addition à un acide ou une base, pharmaceutiquement acceptables.

3. Composés selon la revendication 1 dans lesquels A représente une chaîne hexaméthylène,
leurs isomères optiques, sous forme pure ou sous forme de mélange,
et leurs sels d'addition à un acide ou une base, pharmaceutiquement acceptables.

4. Composés selon la revendication 1 dans lesquels A représente un groupement de formule : 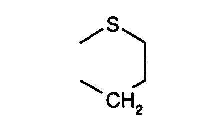 leurs isomères optiques, sous forme pure ou sous forme de mélange,
et leurs sels d'addition à un acide ou une base, pharmaceutiquement acceptables.

5. Composés selon la revendication 1 dans lesquels Ar représente un radical quinolyle,
leurs isomères optiques, sous forme pure ou sous forme de mélange,
et leurs sels d'addition à un acide ou à une base, pharmaceutiquement acceptables.

6. Composés selon la revendication 1 dans lesquels R₃ représente un radical choisi parmi carboxyalkyle et alkoxycarbonylalkyle,
leurs isomères optiques, sous forme pure ou sous forme de mélange,
et leurs sels d'addition à un acide ou une base, pharmaceutiquement acceptables.

7. Composé selon la revendication 1 qui est l'acide 2-{8-[(quinol-2-yl)méthyloxy]thiopyrano[3,2-b]indol-5-yl}acétique,
et ses sels d'addition à un acide ou à une base, pharmaceutiquement acceptables.

8. Composé selon la revendication 1 qui est le 2-(3-[(quinol-2-yl) méthyloxy]-5,6,7,8,9,10,11-heptahydrocyclo-oct[b]indol-11-yl}acétate d'éthyle.

9. Composé selon la revendication 1 qui est l'acide 2-(3-benzyloxy-5,6,7,8,9,10,11-heptahydrocyclo-oct[b]indol-11-yl) acétique et ses sels d'addition avec une base, pharmaceutiquement acceptables.

10. Composé selon la revendication 1 qui est le 2-{3-[(quinol-2-yl)méthyloxy]-5,6,7,8,9,10-hexahydrocyclohept[b]indol-10-yl}acétate d'éthyle.

11. Procédé de préparation des composés de formule (I) selon la revendication 1 caractérisé en ce que l'on condense, en présence d'un amidure de sodium, ou sa combinaison avec un alcoolate (base complexe), le composé de formule (II) : 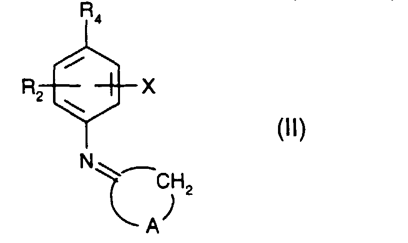 dans laquelle R₂ et A sont tels que définis dans la revendication 1, X représente un atome d'halogène et R₄ représente un radical alkoxy, ou tétrahydropyranyloxy,
pour obtenir :
- soit un composé de formule (III) : 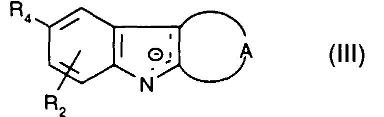 dans laquelle R₂, R₄ et A sont tels que définis précédemment,
- soit, après hydrolyse, un composé de formule (IV) : 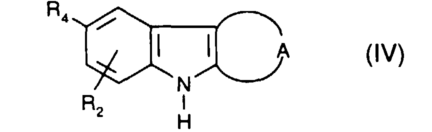 dans laquelle R₂, R₄ et A sont tels que définis précédemment,
composé de formule (III), ou composé de formule (IV) sous forme de son sel alcalin, qui est substitué sur l'azote indolique par un radical de formule R₃', dans lequel R₃' a la même signification que R₃ tel que défini dans la formule (I) à l'exception de l'hydrogène, pour obtenir un composé de formule (V) : 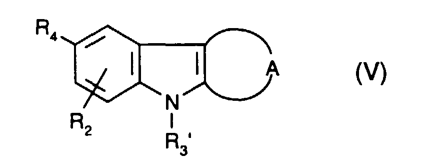 dans laquelle R₂, R₄, A et R₃' sont tels que définis précédemment,
composé de formule (IV) ou (V) qui est soumis à une désalkylation, pour obtenir un composé de formule (VII) : 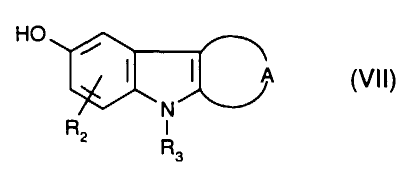 dans laquelle R₂, R₃ et A sont tels que définis précédemment,
composé de formule (VII) qui est mis en réaction avec un composé de formule (VIII) :
Ar-(CH₂)n-Hal'' (VIII)
dans laquelle Ar et n sont tels que définis dans la formule (I) et Hal" représente un halogène,
afin d'obtenir, après une éventuelle saponification pour produire le composé carboxylé à partir de l'ester présent en R₃, le composé de formule (I) 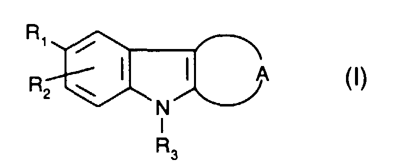 dans laquelle R₁, R₂, R₃ et A sont tels que définis précédemment,
les composés de formule (I) pouvant être, le cas échéant :
- purifiés suivant une ou plusieurs méthodes choisies parmi la cristallisation, la chromatographie sur colonne de silice, l'extraction, la filtration, et le passage sur charbon ou résine,
- séparés, sous forme pure ou sous forme de mélange, en leurs éventuels isomères optiques,
- et transformés, par une base ou un acide, en sels pharmaceutiquement acceptables.

12. Procédé de préparation des composés de formule (I) selon la revendication 1 caractérisé en ce que l'on condense, en présence d'un amidure de sodium ou sa combinaison avec un alcoolate (base complexe), le composé de formule (II') : 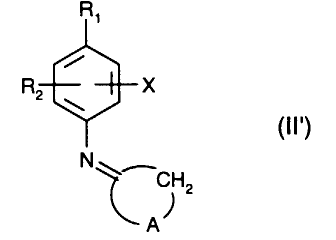 dans laquelle R₁, R₂ et A sont tels que définis dans la formule (I) et X représente un atome d'halogène,
pour obtenir :
- soit un composé de formule (III') : 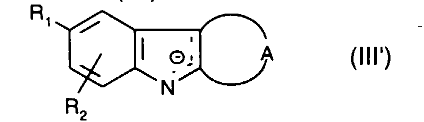 dans laquelle R₁, R₂ et A sont tels que définis précédemment,
- soit, après hydrolyse, un composé de formule (I/a) : 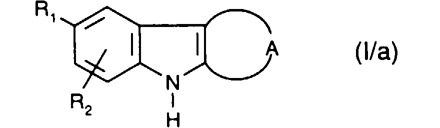 dans laquelle R₁, R₂ et A sont tels que définis précédemment,
composé de formule (III'), ou composé de formule (I/a) sous forme de son sel alcalin, qui est substitué sur l'azote indolique par un radical de formule R₃', dans lequel R₃' a la même signification que R₃ défini dans la revendication 1 à l'exception de l'hydrogène, pour obtenir un composé de formule (I/b) : 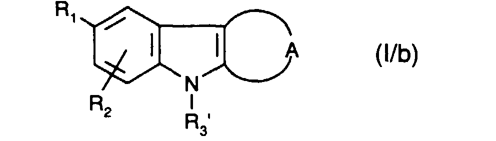 dans laquelle R₁, R₂, A et R₃' sont tels que définis précédemment,
réaction suivie d'une éventuelle saponification pour produire le composé carboxylé à partir de l'ester présent en R₃,
les composés de formule (I/a) et (I/b) formant l'ensemble des composés de formule (I) selon la revendication 1,
les composés de formule (I) selon la revendication 1 pouvant être, le cas échéant :
- purifiés suivant une ou plusieurs méthodes choisies parmi la cristallisation, la chromatographie sur colonne de silice, l'extraction, la filtration, et le passage sur charbon ou résine,
- séparés, sous forme pure ou sous forme de mélange, en leurs éventuels isomères optiques,
- et transformés, par une base ou un acide, en sels pharmaceutiquement acceptables.

13. Compositions pharmaceutiques contenant un des composés de formule (I) selon la revendication 1, ou un de ses sels d'addition à un acide ou à une base, pharmaceutiquement acceptables, seul ou en combinaison avec un ou plusieurs excipients inertes non toxiques.

14. Compositions pharmaceutiques selon la revendication 13 utiles dans le traitement et la prévention de l'inflammation chronique ou aigüe, articulaire, pulmonaire, cutanée, ou rénale et notamment dans la prévention et le traitement de l'arthrite, de la polyarthrite rhumatoïde, de l'ostéo-arthrose, du psoriasis, des maladies allergiques, de l'asthme, des maladies inflammatoires de l'intestin, des ulcères gastro-intestinaux, de l'ischémie, de l'athérosclérose, de la détresse respiratoire et du choc septique.

## Patentansprüche

1. Verbindungen der Formel (I): 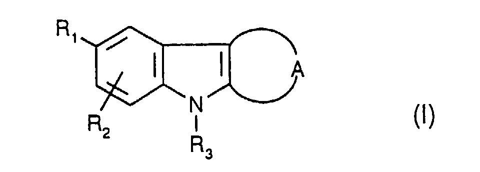 in der:
- R₁ eine Gruppe Ar-(CH₂)ₙ-O-, worin n Null oder eine ganze Zahl mit einem Wert von 1 bis 4 und Ar eine Gruppe ausgewählt aus Phenyl, Naphthyl, Thienyl, Furyl, Pyrrolyl, Imidazolyl, Pyrazolyl, Thiazolyl, Isothiazolyl, Oxazolyl, Isoxazolyl, Thiadiazol, Oxadiazol, Pyridyl, Chinolyl, Isochinolyl, Indolyl, Benzofuryl und Benzothienyl darstellen; worin Ar unsubstituiert oder durch eine oder mehrere Gruppen ausgewählt aus Halogen, Alkyl, Alkoxy, Hydroxy und Trifluormethyl substituiert sein kann,
- R₂ ein Wasserstoffatom oder eine Gruppe ausgewählt aus Halogen, Alkyl, Alkoxy und Trifluormethyl,
- R₃ eine Gruppe ausgewählt aus Wasserstoff, Alkyl. Carboxyalkyl und Alkoxycarbonylalkyl,
- A eine Alkylenkette -(CH₂)ₘ-, worin m zwischen 3 und 6 liegt, oder eine Gruppe der Formel (α): 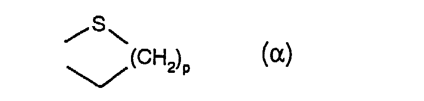 worin p zwischen 1 und 4 liegt,
mit der Maßgabe bedeuten, daß die Begriffe "Alkyl" und "Alkoxy" geradkettige oder verzweigte Gruppen mit 1 bis 6 Kohlenstoffatomen darstellen,
und mit der Maßgabe, daß die Verbindung der Formel (I) weder 7-Benzyloxy-1,2,3,4-tetrahydrocyclopenta[b]indol, 7-Benzyloxy-4*H*-2,3-dihydrothieno[3,2-b]indol, 6-Benzyloxy-1,2,3,4-tetrahydrocarbazol noch 6-Benzyloxy-9-methyl- 1,2,3,4-tetrahydrocarbazol ist,
deren optische Isomeren in reiner Form oder in Form einer Mischung, und deren Additionssalze mit einer pharmazeutisch annehmbaren Säure oder Base.

2. Verbindungen nach Anspruch 1, worin A eine Pentamethylenkette darstellt, deren optische Isomeren in reiner Form oder in Form einer Mischung und deren Additionssalze mit einer pharmazeutisch annehmbaren Säure oder Base.

3. Verbindungen nach Anspruch 1, worin A eine Hexamethylenkette darstellt, deren optische Isomeren in reiner Form oder in Form einer Mischung und deren Additionssalze mit einer pharmazeutisch annehmbaren Säure oder Base.

4. Verbindungen nach Anspruch 1, worin A eine Gruppe der Formel darstellt: 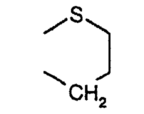 deren optische Isomeren in reiner Form oder in Form einer Mischung und deren Additionssalze mit einer pharmazeutisch annehmbaren Säure oder Base.

5. Verbindungen nach Anspruch 1, worin Ar eine Chinolylgruppe darstellt, deren optische Isomeren in reiner Form oder in Form einer Mischung und deren Additionssalze mit einer pharmazeutisch annehmbaren Säure oder Base.

6. Verbindungen nach Anspruch 1, worin R₃ eine Gruppe ausgewählt aus Carboxyalkyl und Alkoxycarbonylalkyl darstellt,
deren optische Isomeren in reiner Form oder in Form einer Mischung und deren Additionssalze mit einer pharmazeutisch annehmbaren Säure oder Base.

7. Verbindung nach Anspruch 1, nämlich 2-{8-[(Chinol-2-yl)-methyloxy]-thiopyrano[3,2-b]indol-5-yl}-essigsäure.
und deren Additionssalze mit einer pharmazeutisch annehmbaren Säure oder Base.

8. Verbindung nach Anspruch 1, nämlich 2-{3-[(Chinol-2-yl)-methyloxy]-5,6,7,8,9,10,11-heptahydrocyclo-oct[b]indol-11-yl}-essigsäureethylester.

9. Verbindung nach Anspruch 1, nämlich 2-(3-Benzyloxy-5,6,7,8,9,10,11-heptahydrocyclo-oct[b]indol-11-yl)-essigsäure und deren Additionssalze mit einer pharmazeutisch annehmbaren Base.

10. Verbindung nach Anspruch 1, nämlich 2-{3-[(Chinol-2-yl)-methyloxy]-5,6,7,8,9,10-hexahydrocyclohept[b]indol-10-yl}-essigsäureethylester.

11. Verfahren zur Herstellung der Verbindungen der Formel (I) nach Anspruch 1, dadurch gekennzeichnet, daß man die Verbindung der Formel (II): 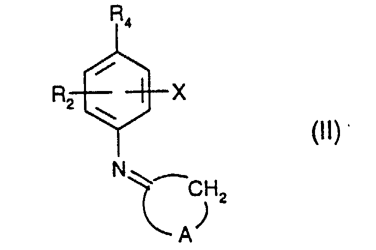 in der R₂ und A die in Anspruch 1 angegebenen Bedeutungen besitzen, X ein Halogenatom darstellt und R₄ eine Alkoxy- oder Tetrahydropyranyloxygruppe bedeutet, in Gegenwart eines Natriumamids oder dessen Kombination mit einem Alkoholat (Basenkomplex) kondensiert, so daß man:
- entweder eine Verbindung der Formel (III) erhält: 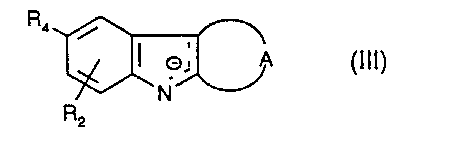 in der R₂, R₄ und A die oben angegebenen Bedeutungen besitzen,
- oder nach der Hydrolyse eine Verbindung der Formel (IV) erhält: 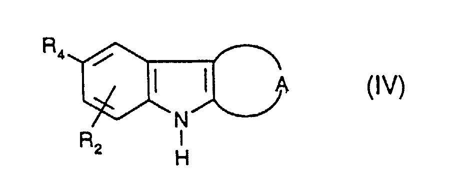 in der R₂, R₄ und A die oben angegebenen Bedeutungen besitzen, welche Verbindung der Formel (III) oder Verbindung der Formel (IV) in Form des Alkalisalzes am Indol-Stickstoffatom mit einer Gruppe der Formel R₃', worin R₃' die Bedeutungen von R₃ wie sie bezüglich der Formel (I) angegeben sind, mit Ausnahme von Wasserstoff, besitzt, substituiert wird, zur Bildung einer Verbindung der Formel (V): 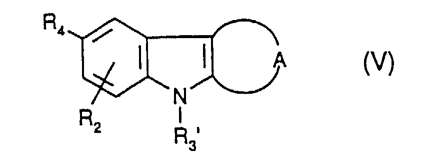 in der R₂, R₄, A und R₃' die oben angegebenen Bedeutungen besitzen, welche Verbindung der Formel (IV) oder (V) einer Desalkylierung unterworfen wird zur Bildung einer Verbindung der Formel (VII): 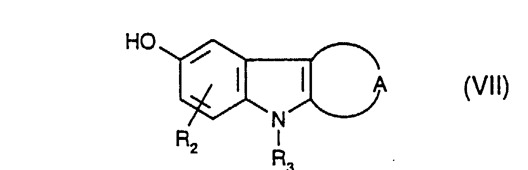 in der R₂, R₃ und A die oben angegebenen Bedeutungen besitzen, welche Verbindung der Formel (VII) mit einer Verbindung der Formel (VIII):
Ar - (CH₂)ₙ - Hal" (VIII)
in der Ar und n die bezüglich der Formel (I) angegebenen Bedeutungen besitzen und Hal" ein Halogen bedeutet, umgesetzt wird, so daß man nach einer eventuellen Verseifung zur Bildung der Carboxylverbindung ausgehend von dem in R₃ vorhandenen Ester die Verbindung der Formel (I) erhält: 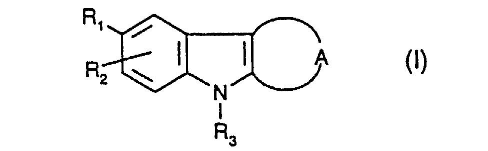 in der R₁, R₂, R₃ und A die oben angegebenen Bedeutungen besitzen, welche Verbindungen der Formel (I) gegebenenfalls:
- gemäß einer oder mehrerer Methoden ausgewählt aus Kristallisation, Säulenchromatographie über Siliciumdioxid, Extraktion, Filtration und Überführung über Aktivkohle oder ein Harz gereinigt werden,
- in deren eventuelle optische Isomeren in reiner Form oder in Form einer Mischung getrennt werden, und
- mit einer Base oder Säure in die pharmazeutisch annehmbaren Salze umgewandelt werden.

12. Verfahren zur Herstellung der Verbindungen der Formel (I) nach Anspruch 1, dadurch gekennzeichnet, daß man die Verbindung der Formel (II'): 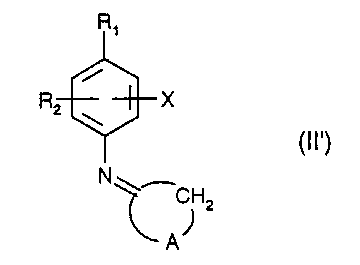 in der R₁, R₂ und A die bezüglich der Formel (I) angegebenen Bedeutungen besitzen und X ein Halogenatom darstellt,
in Gegenwart eines Natriumamids oder einer Kombination davon mit einem Alkoholat (Basenkomplex) kondensiert, so daß man:
- entweder eine Verbindung der Formel (III') erhält: 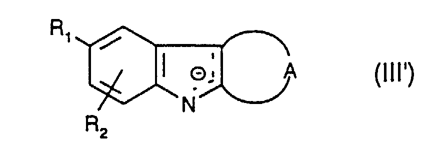 in der R₁, R₂ und A die oben angegebenen Bedeutungen besitzen,
- oder nach der Hydrolyse eine Verbindung der Formel (I/a) erhält: 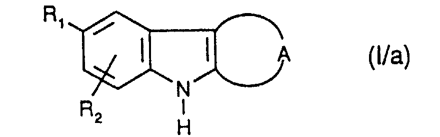 in der R₁, R₂ und A die oben angegebenen Bedeutungen besitzen, welche Verbindung der Formel (III') oder Verbindung der Formel (I/a) in Form des Alkalisalzes am Indol-Stickstoff mit einer Gruppe der Formel R₃', worin R₃' die Bedeutungen von R₃, wie sie in Anspruch 1 definiert sind, mit Ausnahme von Wasserstoff, besitzt, zur Bildung einer Verbindung der Formel (I/b) substituiert wird: 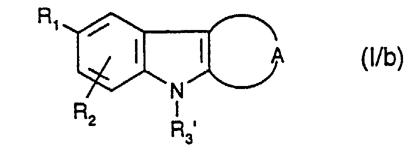 in der R₁, R₂, A und R₃' die oben angegebenen Bedeutungen besitzen, welche Reaktion gegebenenfalls von einer Verseifung gefolgt wird zur Bildung der Carboxylverbindung ausgehend von dem in R₃ vorhandenen Ester, wobei die Verbindungen der Formeln (I/a) und (I/b) die Gesamtheit der Verbindungen der Formel (I) gemäß Anspruch 1 bilden,
welche Verbindungen der Formel (I) nach Anspruch 1 gegebenenfalls:
- gemäß einer oder mehrerer Methoden ausgewählt aus Kristallisation, Säulenchromatographie über Siliciumdioxid, Extraktion, Filtration und Überführung über Aktivkohle oder ein Harz gereinigt wird,
- in deren eventuelle optische Isomeren in reiner Form oder in Form einer Mischung getrennt werden, und
- mit einer Base oder Säure in die pharmazeutisch annehmbaren Salze umgewandelt werden.

13. Pharmazeutische Zubereitungen enthaltend eine der Verbindungen der Formel (I) nach Anspruch 1 oder eines ihrer Additionssalze mit einer pharmazeutisch annehmbaren Säure oder Base allein oder in Kombination mit einem oder mehreren inerten, nichttoxischen Trägermaterialien.

14. Pharmazeutische Zubereitungen nach Anspruch 13 zur Behandlung und zur Vorbeugung von chronischen oder akuten Gelenk-, Lungen-, Haut- oder Nierenentzündungen und insbesondere zur Vorbeugung und Behandlung der Arthritis, der rheumatoiden Polyarthritis, der Osteoarthrose, der Psoriasis, von allergischen Erkrankungen, von Asthma, von Darmentzündungserkrankungen, von Magen-Darm-Geschwüren, von Ischämie, von Atherosklerose, von Atemnot und septischem Schock.

## Claims

1. Compounds of formula (I): 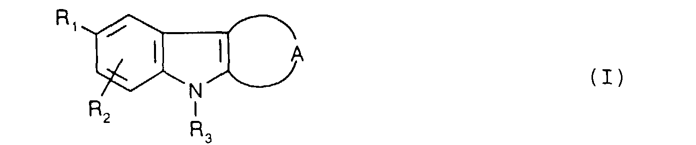 in which:
- R₁ represents a group Ar-(CH₂)ₙ-O- wherein n represents zero or an integer from 1 to 4 and Ar is a radical selected from phenyl, naphthyl, thienyl, furyl, pyrrolyl, imidazolyl, pyrazolyl, thiazolyl, isothiazolyl, oxazolyl, isoxazolyl, thiadiazole, oxadiazole, pyridyl, quinolyl, isoquinolyl, indolyl, benzofuryl and benzothienyl, Ar being unsubstituted or substituted by one or more radicals selected from halogen, alkyl, alkoxy, hydroxy and trifluoromethyl;
- R₂ represents a hydrogen atom or a radical selected from halogen, alkyl, alkoxy and trifluoromethyl;
- R₃ represents a radical selected from hydrogen, alkyl, carboxyalkyl and alkoxycarbonylalkyl; and
- A represents an alkylene chain -(CH₂)ₘ- wherein m is from 3 to 6, or represents a group of formula (α): 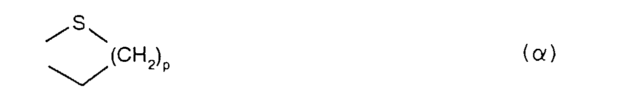 in which p is from 1 to 4;
wherein the terms "alkyl" and "alkoxy" denote linear or branched groups having from 1 to 6 carbon atoms,
wherein the compound of formula (I) may not be 7-benzyloxy-1,2,3,4-tetrahydrocyclopenta [b]indole, 7-benzyloxy-4H-2,3-dihydrothieno[3,2-b]indole, 6-benzyloxy-1,2,3,4-tetrahydrocarbazole or 6-benzyloxy-9-methyl-1,2,3,4-tetrahydrocarbazole,
their optical isomers, in pure form or in the form of a mixture,
and pharmaceutically acceptable addition salts thereof with an acid or a base.

2. Compounds according to claim 1 in which A represents a pentamethylene chain,
their optical isomers, in pure form or in the form of a mixture,
and pharmaceutically acceptable addition salts thereof with an acid or a base.

3. Compounds according to claim 1 in which A represents a hexamethylene chain,
their optical isomers, in pure form or in the form of a mixture,
and pharmaceutically acceptable addition salts thereof with an acid or a base.

4. Compounds according to claim 1 in which A represents a group of the formula: 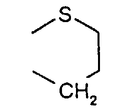 their optical isomers, in pure form or in the form of a mixture,
and pharmaceutically acceptable addition salts thereof with an acid or a base.

5. Compounds according to claim 1 in which Ar represents a quinolyl radical,
their optical isomers, in pure form or in the form of a mixture,
and pharmaceutically acceptable addition salts thereof with an acid or a base.

6. Compounds according to claim 1 in which R₃ represents a radical selected from carboxyalkyl and alkoxycarbonylalkyl,
their optical isomers, in pure form or in the form of a mixture,
and pharmaceutically acceptable addition salts thereof with an acid or a base.

7. Compound according to claim 1 that is 2-{8-[(quinol-2-yl)methyloxy]thiopyrano[3,2-b]indol-5-yl}acetic acid, and pharmaceutically acceptable addition salts thereof with an acid or a base.

8. Compound according to claim 1 that is ethyl 2-{3-[(quinol-2-yl)methyloxy]-5,6,7,8,9,10,11-heptahydrocyclooct[b]indol-11-yl}acetate.

9. Compound according to claim 1 that is 2-(3-benzyloxy-5,6,7,8,9,10,11-heptahydrocyclooct[b]indol-11-yl)acetic acid, and pharmaceutically acceptable addition salts thereof with a base.

10. Compound according to claim 1 that is ethyl 2-{3-[(quinol-2-yl)methyloxy]-5,6,7,8,9,10-hexahydrocyclohept[b]indol-10-yl}acetate.

11. Process for the preparation of compounds of formula (I) according to claim 1, characterised in that a compound of formula (II): 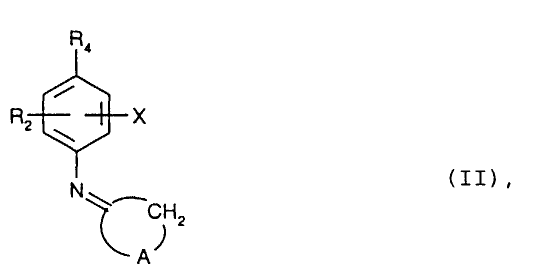 in which R₂ and A are as defined in claim 1, X represents a halogen atom and R₄ represents an alkoxy radical, or tetrahydropyranyloxy, is condensed in the presence of sodium amide or a combination thereof with an alcoholate (complex base) to obtain:
- either a compound of formula (III): 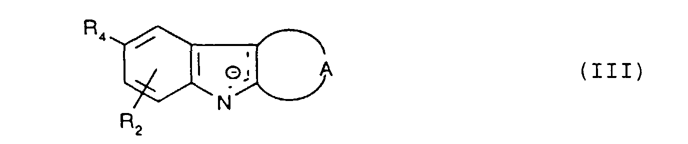 in which R₂, R₄ and A are as defined above,
- or, after hydrolysis, a compound of formula (IV): 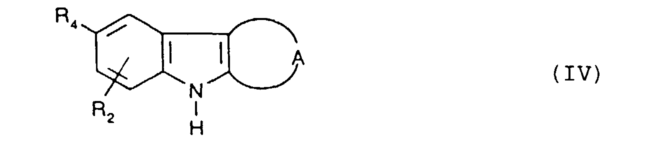 in which R₂, R₄ and A are as defined above,
which compound of formula (III), or compound of formula (IV) in the form of its alkali metal salt, is substituted on the indole nitrogen by a radical of the formula R₃', wherein R₃' has the same meanings as R₃ in the definition of formula (I) with the exception of hydrogen, to obtain a compound of formula (V): 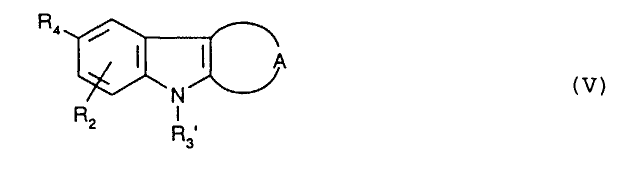 in which R₂, R₄, A and R₃' are as defined above, which compound of formula (IV) or (V) is subjected to dealkylation to obtain a compound of formula (VII): 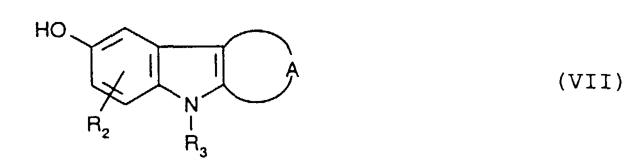 in which R₂, R₃ and A are as defined above,
which compound of formula (VII) is reacted with a compound of formula (VIII):
Ar-(CH₂)ₙ-Hal" (VIII)
in which Ar and n are as defined in formula (I) and Hal" represents a halogen atom,
to obtain, optionally after hydrolysis to produce the carboxylated compound from the ester present in R₃, the compound of formula (I): 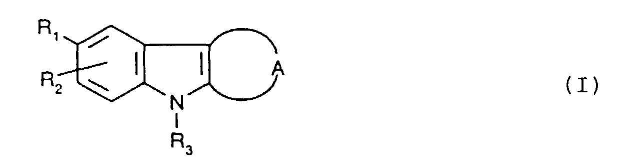 in which R₁, R₂, R₃ and A are as defined above, which compounds of formula (I) may optionally be:
- purified by one or more methods selected from crystallisation, chromatography on a silica column, extraction, filtration, and passage over charcoal or resin,
- separated, in pure form or in the form of a mixture, into their possible optical isomers,
- and converted into pharmaceutically acceptable salts with a base or an acid.

12. Process for the preparation of compounds of formula (I) according to claim 1, characterised in that a compound of formula (II'): 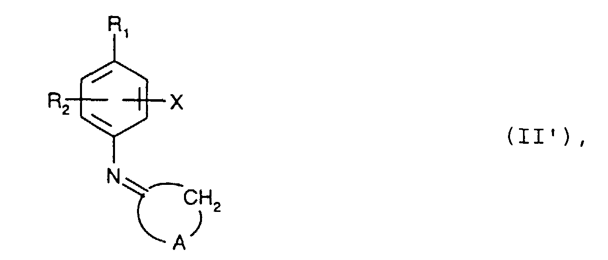 in which R₁, R₂ and A are as defined in formula (I) and X represents a halogen atom,
is condensed in the presence of sodium amide or a combination thereof with an alcoholate (complex base) to obtain:
- either a compound of formula (III'): 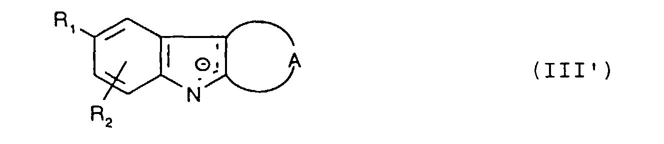 in which R₁, R₂ and A are as defined above,
- or, after hydrolysis, a compound of formula (I/a): 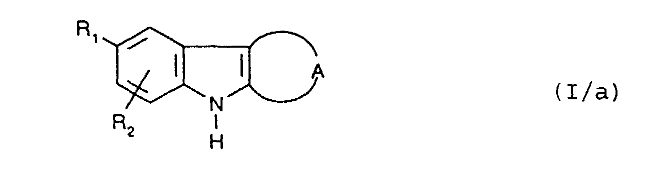 in which R₁, R₂ and A are as defined above,
which compound of formula (III'), or compound of formula (I/a) in the form of its alkali metal salt, is substituted on the indole nitrogen by a radical of the formula R₃', wherein R₃' has the same meanings as R₃ in the definition given in claim 1 with the exception of hydrogen, to obtain a compound of formula (I/b): 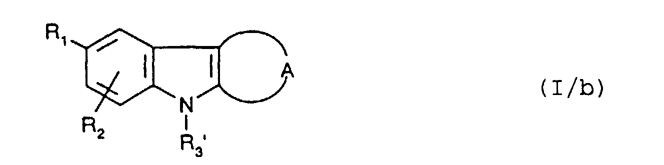 in which R₁, R₂, A and R₃' are as defined above,
which reaction is optionally followed by hydrolysis to produce the carboxylated compound from the ester present in R₃,
the compounds of formulae (I/a) and (I/b) forming the totality of the compounds of formula (I) according to claim 1,
which compounds of formula (I) according to claim 1 may optionally be:
- purified by one or more methods selected from crystallisation, chromatography on a silica column, extraction, filtration, and passage over charcoal or resin,
- separated, in pure form or in the form of a mixture, into their possible optical isomers,
- and converted into pharmaceutically acceptable salts with a base or an acid.

13. Pharmaceutical compositions comprising one of the compounds of formula (I) according to claim 1 or a pharmaceutically acceptable addition salt thereof with an acid or a base, on its own or in combination with one or more inert, non-toxic excipients.

14. Pharmaceutical compositions according to claim 13 for use in the treatment and prevention of chronic or acute, articular, pulmonary, cutaneous or renal inflammation, and especially in the prevention and treatment of arthritis, rheumatoid polyarthritis, osteoarthrosis, psoriasis, allergic disorders, asthma, inflammatory diseases of the intestine, gastrointestinal ulcers, ischaemia, atherosclerosis, respiratory distress and septic shock.
